# EUROPEAN PATENT APPLICATION

(11) **EP 3 446 724 A1**
(43) Date of publication of application: **27.02.2019**
(21) Application number: 18189992.3
(22) Date of filing: 21.08.2018
(51) Int. Cl.: A61L 29/08, A61L 29/12, A61F 2/958, A61M 25/10, B82Y 30/00

(54) **MEDICAL BALLOONS WITH REINFORCED NANOCOMPOSITE MATERIALS AND METHOD OF MAKING THE SAME**

(30) Priority: 22.08.2017 US 201762548538 P
(71) Applicant: Cook Medical Technologies LLC, Bloomington, IN 47404 (US)
(72) Inventor: MOELLER, Rasmus Buch, 4100 Ringsted (DK)
(74) Representative: Garratt, Peter Douglas

(57) **Abstract**

A medical device includes an inflatable balloon having an axial length with a wall located along the axial length having an interior surface and an exterior surface that defines a wall thickness there between. At least a portion of the wall is formed of a reinforced nanocomposite that includes a polymeric matrix and a plurality of nanofibers. The nanofibers are aligned in a direction that ranges from being circumferentially-oriented and/or axially-oriented with respect to the axial length of the inflatable balloon.

## Description

### FIELD

This disclosure relates generally to medical balloons reinforced with nanocomposite materials, the use of such reinforced medical balloons, and a method of fabricating the reinforced medical balloons.

### BACKGROUND

The statements in this section merely provide background information related to the present disclosure and may not constitute prior art.

The body includes a variety of passageways in the form of arteries and other blood vessels. A passageway can sometimes become constricted or blocked by the accumulation of plaque or the growth of a tumor. When this occurs, the constricted passageway needs to be expanded in an angioplasty procedure using a balloon catheter, which includes a medical balloon carried on a catheter shaft.

Balloon angioplasty has become a widely used procedure for expanding constricted passageways. In the angioplasty procedure, a catheter delivers an uninflated medical balloon to the constricted region in the passageway. After positioning the balloon in the passageway, fluid injected through the catheter's lumen into the balloon causes the balloon to inflate and exert pressure against the constricted region to expand the passageway. After use, the balloon is collapsed and withdrawn with the catheter.

During use, medical balloons must expand in a predictable manner and withstand exposure to very high pressures that force the surface of the balloon against various vessel tissues and deposits that exhibit a variety of viscoelastic characteristics and may include some hard and/or rough surfaces. In this sense, the balloons must exhibit high strength and be puncture resistant. In addition, the balloons must also be thin-walled in order to collapse into a small cross-sectional profile necessary for introduction to and removal from the constricted passageway via a catheter.

### SUMMARY

The present disclosure generally provides a medical device that comprises an inflatable balloon having an axial length with a wall located along the axial length having an interior surface and an exterior surface that defines a wall thickness there between; at least a portion of the wall being formed of a reinforced nanocomposite. The reinforced nanocomposite comprises a polymeric matrix and a plurality of nanofibers; wherein the nanofibers are aligned circumferentially and/or axially with respect to the axial length of the inflatable balloon.

According to another aspect of the present disclosure, a method of forming a medical device comprising an inflatable balloon having an axial length with a wall located along the axial length having an interior surface and an exterior surface that defines a wall thickness there between is provided. The method comprises providing a polymeric matrix; providing a plurality of nanofibers; dispersing the plurality of nanofibers into the polymeric matrix to form a reinforced nanocomposite material; forming the inflatable balloon from the reinforced nanocomposite material using an extrusion process, a blow molding process, an injection molding process, a compression molding process, or a sheet-forming process; and aligning the nanofibers circumferentially and/or axially with respect to the axial length of the inflatable balloon.

According to yet another aspect of the present disclosure, the use of a reinforced nanocomposite material to form a medical device is provided wherein the medical device includes an inflatable balloon having an axial length with a wall located along the axial length having an interior surface and an exterior surface that defines a wall thickness there between. At least a portion of the wall is formed of the reinforced nanocomposite material. The reinforced nanocomposite material comprises a polymeric matrix; and a plurality of nanofibers; wherein the nanofibers are aligned circumferentially and/or axially with respect to the axial length of the inflatable balloon.

Further areas of applicability will become apparent from the description provided herein. It should be understood that the description and specific examples are intended for purposes of illustration only and are not intended to limit the scope of the present disclosure.

### DRAWINGS

In order that the disclosure may be well understood, there will now be described various forms thereof, given by way of example, reference being made to the accompanying drawings, in which:
Figure 1A is a perspective view of an inflatable balloon formed from a reinforced nanocomposite material with nanofibers aligned in a circumferential direction according to the teachings of the present disclosure;
Figure 1B is a cross-sectional view of the inflatable balloon of Figure 1A taken along plane (a);
Figure 2A is a perspective view of another inflatable balloon formed from a reinforced nanocomposite material with nanofibers aligned in an axial direction according to the teachings of the present disclosure;
Figure 2B is a cross-sectional view of the inflatable balloon of Figure 2A taken along plane (a);
Figure 3A is a perspective view of another inflatable balloon formed from a reinforced nanocomposite material with nanofibers aligned biaxially according to the teachings of the present disclosure;
Figure 3B is a cross-sectional view of the inflatable balloon of Figure 3A taken along plane (a);
Figure 3C is a cross-section view of another inflatable balloon formed according to the teachings of the present disclosure in which the reinforced nanocomposite material is a coating applied the external surface of a polymer layer;
Figure 4A a perspective view of a comparable inflatable balloon formed from a comparative nanocomposite material with nanofibers aligned in a radial direction;
Figure 4B is a cross-sectional view of the inflatable balloon of Figure 3A taken along plane (a);
Figure 5 is a cross-sectional view of a segment of another inflatable balloon formed according to the teachings of the present disclosure illustrating the use of multiple layers;
Figure 6A is a top-down view of a section of a reinforced nanocomposite material showing the alignment of the nanofibers in the polymeric matrix according to the teachings of the present disclosure;
Figure 6B is a top-down view of a section of another reinforced nanocomposite material showing biaxial alignment of the nanofibers in the polymeric matrix according to the teachings of the present disclosure;
Figure 7 is a flowchart describing a method of manufacturing a medical device having an inflatable medical balloon formed of a reinforced nanocomposite material according to the teachings of the present disclosure; and

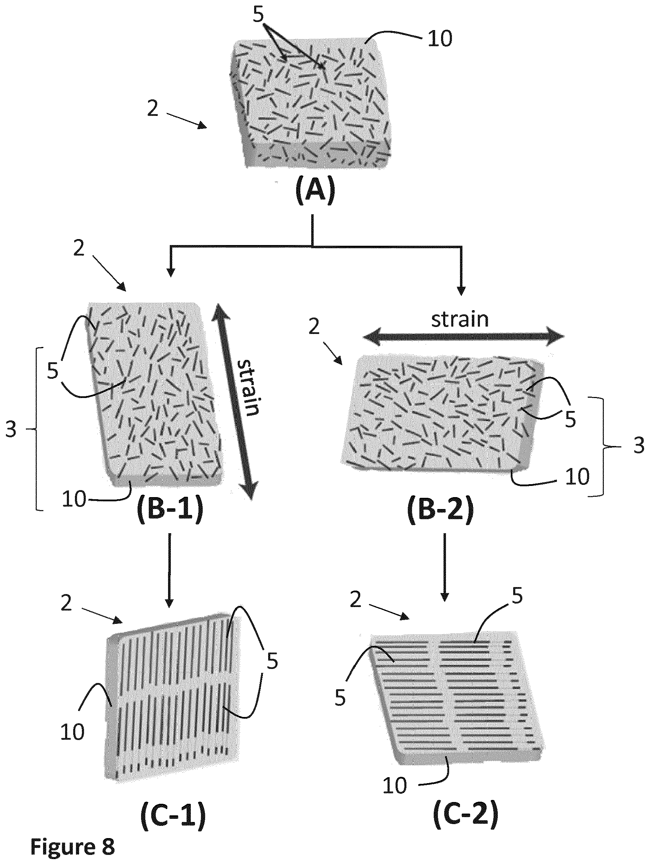

Figure 8 is a schematic representation of a method of aligning nanofibers dispersed in an polymeric matrix of a reinforced nanocomposite material in a circumferential and/or axial direction.

The drawings described herein are for illustration purposes only and are not intended to limit the scope of the present disclosure in any way.

### DETAILED DESCRIPTION

The following description is merely exemplary in nature and is in no way intended to limit the present disclosure or its application or uses. For example, the reinforced nanocomposite materials made and used according to the teachings contained herein is described throughout the present disclosure in conjunction with thin tubular balloons used in angioplasty procedures performed in coronary vessels in order to more fully illustrate the composition and the use thereof. The incorporation and use of such reinforced nanocomposite materials in medical balloons used in other applications, including valvuloplasty, minimally invasive repair of heart valves, the repair or removal of aneurysms, the deployment of stents in coronary and carotid arteries and other peripheral vessels, or the like are contemplated to be within the scope of the present disclosure. It should be understood that throughout the description, corresponding reference numerals indicate like or corresponding parts and features.

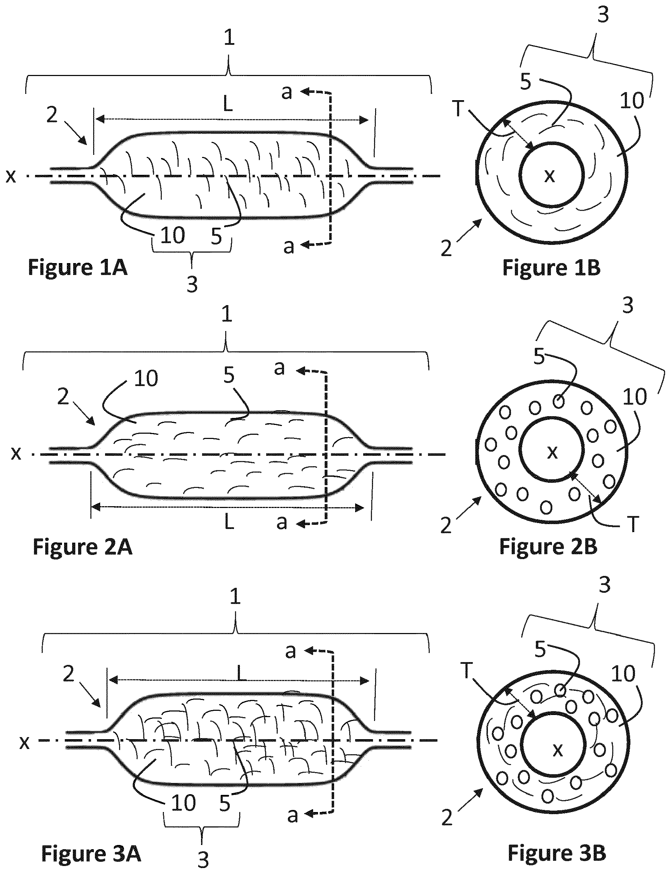

Referring to Figures 1A, 1B, 2A, 2B, 3A, & 3B the present disclosure generally provides a medical device 1 that comprises an inflatable balloon 2 having a length (L) along axis (X) with a wall located along the axial length having an interior surface and an exterior surface that defines a wall thickness (T) there between. At least a portion of the wall is formed of a reinforced nanocomposite 3. The reinforced nanocomposite 3 comprises a polymeric matrix 10 and a plurality of nanofibers 5, wherein the nanofibers 5 are aligned circumferentially (shown in Figures 1A & 1B), axially (shown in Figures 2A & 2B), and/or biaxially with nanofibers arranged in at least two directions ranging from an axial to circumferential direction (shown in Figures 3A & 3B) with respect to axis (X), e.g., along the axial length (L) of the inflatable balloon.

The use of aligned nanofibers dispersed in a polymeric matrix can modify properties, such as the toughness, tensile strength, modulus, and elongation, among other properties of the reinforced nanocomposite material, thereby, providing a more reliable product that is less prone to breakage. In addition, the inflatable balloons formed using reinforced nanocomposite materials provides for a higher burst pressure or lower balloon profile. The reinforced nanocomposite materials of the present disclosure allow for the use of non-conventional balloon materials due to the combined effect of the properties associated with the polymeric matrix and the dispersed and aligned nanofibers. The reinforcing effect provided by the aligned nanofibers in the nanocomposite materials is not limited to the final manufactured medical device, but rather such a reinforcing effect encountered during the manufacturing process reduces the rate of defect formation, thereby, resulting in higher yield and lower scrap costs.

The ability to modify the properties of a medical balloon allows for customization of balloons for use in specific applications. The modification of these properties can result in an increase or decrease in the targeted property as desired or necessary for use of an inflatable balloon in a predetermined application. For example, if a stronger more compliant balloon is desired, adding "soft" (e.g., not to rigid) or compliant nanofibers to the polymeric matrix will increase tensile strength and elongation without substantially affecting the elastic modulus of the inflatable balloon. Similarly, if a stronger less compliant balloon is desired, the use of rigid nanofibers can increase the tensile strength and modulus, while decreasing elongation. Thus, the ability to modify the properties associated with the reinforced nanocomposite materials allows for the use of unconventional materials in the formation of an inflatable balloon incorporated into a medical device.

Referring once again to Figures 1A, 1B, 2A, 2A, 3A, & 3B, the nanofibers 5 dispersed throughout the polymeric matrix 10 in the reinforced nanocomposite materials 3 represent thin short strands, tubes, or wires of various lengths that are relatively linear in geometry. In other words, the nanofibers 5 are aligned, such that they extend in at least one direction relative to the axis (X) that corresponds to the axial length (L) of the inflatable balloon 2 in the medical device 1. Referring now to Figures 1A and 1B, the nanofibers 5 may be aligned in a circumferential direction relative to axis (X). Alternatively, the nanofibers 5 may be aligned in an axial direction relative to axis (X) or the axial length (L) of the inflatable balloon 2. Alternatively, the nanofibers 5 may be aligned biaxially in two or more directions that range from the axial to circumferential directions relative to axis (X). Thus, the nanofibers 5 therefore provide for the improvement of properties in a direction e.g. circumferentially or axially, as opposed to radially. In other words, the inflatable balloon 2 may be compliant in one direction and not in the other direction. Similarly, the strength exhibited by the inflatable balloon 2 in one direction may be increased without doing so in the transverse direction.

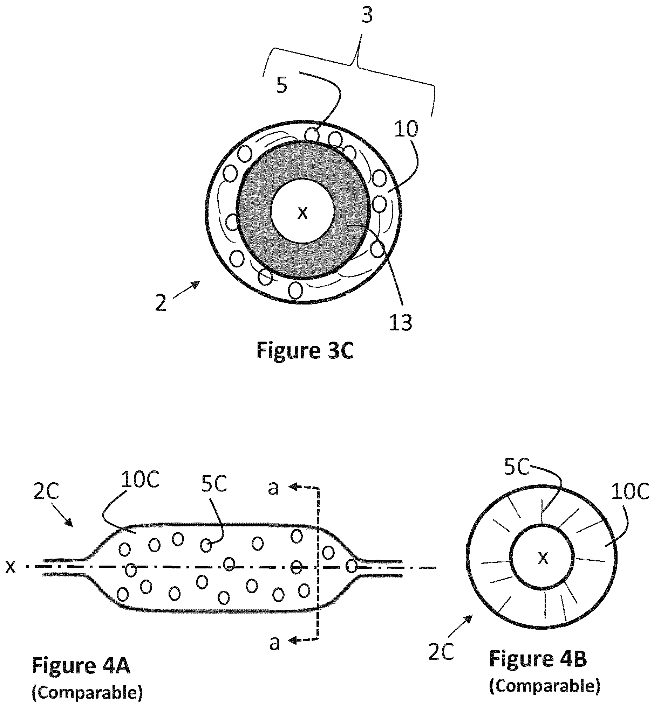

Referring now to Figure 3C, the inflatable balloon 2 may comprise an inner layer of a polymeric material 13. In this case, the reinforced nanocomposite material 3 comprising the polymeric matrix 10 and the nanofibers 5 is applied as a coating or outer layer onto the external surface of the inner polymer layer. The composition of the inner polymer layer may be the same or different from the composition of the polymeric matrix present in the reinforced nanocomposite material as further defined herein.

A comparative example is provided in Figures 4A and 4B in which nanofibers 5C dispersed in a polymeric matrix 10C are arranged in a radial direction relative to axis (X) or along the axial length (L) of an inflatable balloon 2C. Alignment of nanofibers in a radial direction is generally unlikely, and/or undesirable.

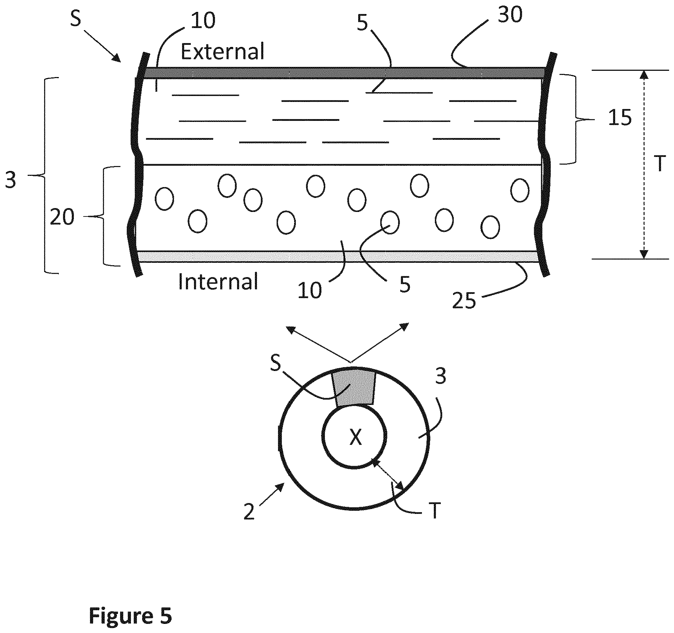

According to another aspect of the present disclosure, the reinforced nanocomposite material may comprise two or more layers. Referring now to Figure 5, a section (S) of a nanocomposite material 3 in an inflatable balloon 2 having a wall thickness (T) is described. In this case, the reinforced nanocomposite material 3 is shown to comprise two layers 15, 20 in which the nanofibers 5 are oriented in different directions relative to axis (X). In this specific example, the nanofibers 5 in one layer 20 are axially oriented, while the nanofibers 5 in the other layer 15 are circumferentially oriented. Alternatively, the reinforced nanocomposite material may comprise more than 2 layers when desirable. The nanofibers 5 and polymeric matrix 10 in each of the layers 15, 20 may comprise the same or different composition of materials.

Optionally, the reinforced nanocomposite material 3 may comprise at least one of an inner protective layer 25 and an outer protective layer 30. The protective layers 25, 30 do not need to be reinforced, but can be if so desired. These protective layers 25, 30 are individually selected to comprise the same material composition or a different composition than the polymeric matrix 10 of the reinforced nanocomposite material 3.

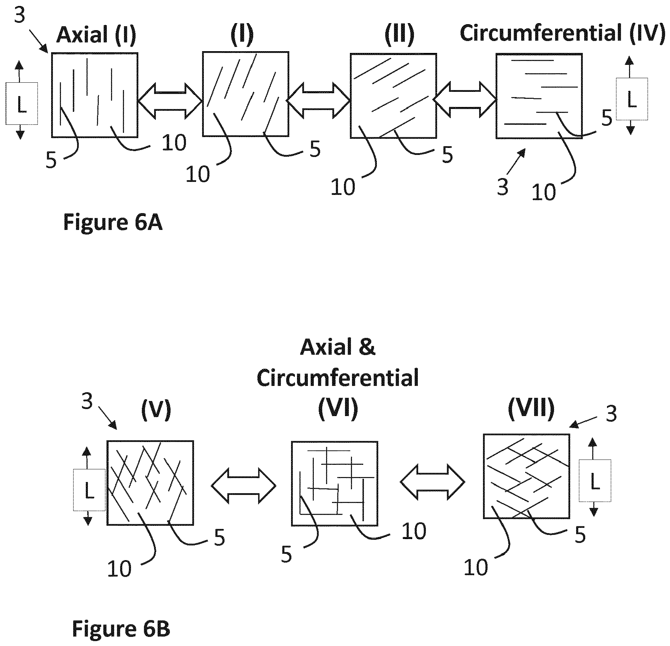

According to another aspect of the present disclosure, the orientation of the nanofibers in the polymeric matrix of the reinforced nanocomposite material of the inflatable medical balloon are arranged in a predetermined pattern. This predetermined pattern may range from the nanofibers being circumferentially-oriented to axially-oriented or at any angle therebetween relative to the axial length of the inflatable balloon. Referring now to Figures 6A & 6B a top-down view of a section of the reinforced nanocomposite material 3 is shown with nanofibers 5 dispersed in a polymeric matrix 10 and aligned axially (I) or circumferentially (IV) to the axial length (L) of the inflatable balloon or biaxially in both an axial and circumferential direction (VI). When desirable or necessary to achieve a level of performance that is intermediate between the reinforced nanocomposites with the nanofibers axially (I) and circumferentially (IV) oriented, a nanocomposite can be formed and used in which the nanofibers are oriented at an angle that falls there between as depicted in (II) and (III) of Figure 6A. Similarly, biaxially aligned nanofibers (VI) may be aligned at an intermediate angle that falls within the axial and circumferential directions as depicted in (V) and (VII) of Figure 6B. Alternatively, the nanofibers 5 dispersed in the polymeric matrix 10 are aligned at an intermediate angle between the extremes of axial-orientation and circumferentialorientation.

Some medical balloons are required to perform under conditions wherein extensions greater than 100% are experienced. Upon stretching a medical balloon, the resulting extension in length is accompanied by a thinning of the cross-sectional area or wall thickness. Since the reinforcing fibers usually are more rigid than the polymeric matrix, the reduction in wall thickness that occurs upon extension does not result in a proportional reduction in the thickness of the reinforcing fibers. Thus, the use of a fiber that is larger in size than the reduced wall thickness may cause distortions, exhibit reduced resistance to breakage, and provide points for potential failure. In other words, the use of reinforcing fibers in the reinforced nanocomposite materials in the present disclosure is limited to nanofibers.

As used herein the term "nanofiber" is meant to include, but not be limited to nanotubes, nanowires, nanobelts, nanofibrils, fiber bundles, or the like, including without limitation single and multi-walled nanotubes. Each nanofiber is individually selected, such that a mixture of different nanofibers may be included in the reinforced nanocomposite materials. The nanofibers are incorporated into the polymeric matrix to form a reinforced nanocomposite material in an amount that is between about 0.1 wt.% to less than 5 wt.%, based on the overall weight of the reinforced nanocomposite material. Alternatively, the amount of nanofibers incorporated into the reinforced nanocomposite materials is less than about 3 wt.%, based on the overall weight of the reinforced nanocomposite material. Alternatively, the reinforced nanocomposite materials comprise between 0.1 wt. % to 4.0 wt.%, between 0.5 wt.% and 3.0 wt.%, from 1.0 wt.% to 3.0 wt.%, or from 1.0 wt.% to 2.0 wt.% nanofibers, based on the overall weight of the reinforced nanocomposite material.

For the purpose of this disclosure, the term "weight" refers to a mass value, such as having the units of grams, kilograms, and the like. Further, the recitations of numerical ranges by endpoints include the endpoints and all numbers within that numerical range. For example, a concentration ranging from 40% by weight (wt.%) to 60 wt.% by weight includes concentrations of 40% by weight, 60% by weight, and all concentrations there between (e.g., 40.1%, 41%, 45%, 50%, 52.5%, 55%, 59%, etc.).

The nanofibers used in the reinforced nanocomposite materials of the present disclosure may have a diameter of up to 20 nanometers (nm), up to 15 nm, up to 10 nm, or up to 5 nm. Alternatively, the nanofibers have a diameter that is in the range of about 1 nm to about 20 nm, alternatively, from about 3 nm to about 15 nm, from about 3 nm to about 10 nm, or alternatively, from about 2 nm to about 7 nm.

The length of the nanofibers may range from about 5 nanometers (nm) to about 50,000 nm, from about 100 nm to about 7,500 nm, or from about 500 nm to about 6,000 nm. Alternatively, the length of the nanofibers is less than 50,000 nanometers, less than 25,000 nanometers, less than 10,000 nanometers, less than 7,500 nanometers, or less than 6,000 nanometers.

According to another aspect of the present disclosure, the nanofibers used in the reinforced nanocomposite materials exhibit a fiber aspect ratio of at least 25, at least 50, at least 100, or at least 250. Alternatively, the fiber aspect ratio ranges from about 25 to about 10,000, from about 50 to about 5,000, from about 100 to about 1,000, or from about 300 to about 900. As used herein, the term "aspect ratio" refers to the ratio determined by dividing the average length of the nanofibers by the average diameter of the nanofibers.

The nanofibers used in the present disclosure may be made of one or more natural or synthetic materials. According to one aspect of the present disclosure, the nanofibers are biocompatible and comprise one or more synthetic or natural polymeric materials. Several examples of synthetic polymeric materials include, but are not limited to nanofibrillated cellulose (NFC), cellulose nanocrystals (CNC), poly(E-caprolactone) (PCL), poly(lactic acid) (PLA), poly(glycolic acid) (PGA), poly(lactide-co-glycolide) (PLGA), poly(L-lactide) (PLL), poly(L-lactide-co-Ecaprolactone) [P(LLA-CL)], polyurethane (PU), polyethylene oxide (PEa), polyethylene terephthalate (PET), poly (ester urethane)urea (PEUW), poly[bis(p-methylphenoxy) phosphazene] (PNmPh), poly(p-dioxanone-co-L-lactideblock-poly(ethylene glycol) (PPDO/PLLA -b-PEG), and polyamides. Several examples of polymeric natural materials include, without limitation, collagen, gelatin (denatured collagen), elastin, alpha-elastin, tropoelastin, and chitosan.

When desirable or necessary to achieve the properties required for a predetermined application, the nanofibers may also comprise monomeric silicates, such as polyhedral oligomeric silsesquioxanes (POSS); carbon and ceramic materials, such as polyacrylonitrile, single-walled and multi-walled nanotubes, silica nanogels, and metals or metal oxides. These metal or metal oxide nanofibers may include, without limitation alumina (Al₂O₃), titanium oxide (TiO₂), tungsten oxide, zirconium oxide, gold (Au), silver (Ag), and platinum (Pt), as well as nanofibers that include magnetic or paramagnetic materials, such as neodinium iron boron or super paramagnetic ferrite oxide (Fe₃O₄) or super paramagnetic maghemite (Fe₂O₃). The nanofibers may also comprise other organic materials including, without limitation, temperature sensitive polymers, such as polyvinylpyrrolidone and n-isopropylacrylamide copolymers or blends, and poloxamer, biodegradable polymers such as poly(lactic) acid, polysaccharide, and polyalkylcyanoacrylate. Alternatively, the nanofibers comprise nanofibrillated cellulose (NFC), cellulose nanocrystals (CNC), carbon nanotubes, metal oxides, or a combination thereof.

According to another aspect of the present disclosure, the nanofibers may be single-walled nanotubes (SWNT) or multi-walled nanotubes (MWNT). When desirable, the nanotubes may be double-walled nanotubes (DWNT). Several specific examples of nanotubes include, but are not limited to, carbon nanotubes, boron nitride nanotubes, aluminum nitride nanotubes, and gold nanotubes. The nanotubes may be positively charged, negatively charged, or neutral with respect to charge.

According to another aspect of the present disclosure, the nanofibers may comprise cellulose nanocrystals (CNC) or nanofibrillated cellulose (NFC). The nanofibers may be comprised of several or more nanocrystalline elementary fibrils formed by cellulose chains (homopolymers of glucose), concreted by/in a matrix containing lignin, hemicellulose and other components. The nanofibers may comprise consist of, or consist essentially of monocrystalline cellulose domains linked by amorphous domains. The amorphous regions may act as structural defects and can be removed under acid hydrolysis, leaving cellulose rod-like nanocrystals or whiskers, and have a morphology and crystallinity similar to the original cellulose nanofibers.

The nanofibers may be inherently hydrophilic or hydrophobic, or the nanofibers may be made to exhibit hydrophilic or hydrophobic properties by the addition of one or more surface functional groups. Several specific examples of hydrophilic functional groups include, without limitation, hydroxyl groups, carbonyl groups, carboxyl groups, and carboxylate groups. Several specific examples of hydrophobic functional groups include, but are not limited to hydrocarbons, silicones, and fluorocarbons.

Cellulosic materials, such as nanocellulose, in their natural state have a surface comprising a substantial number of hydroxyl (-OH) groups. The presence of these hydroxyl groups provide for the development of a strong interface between the nanocellulose fibers and the polymeric matrix through hydrogen bonding and other attractive forces. Alternatively, nanocellulose fibers with surface chemical functional groups other than hydroxyl groups may be used. Such other functional groups may be incorporated into the nanocellolse fibers using without limitation one or more of TEMPO oxidation, carboxymethylation, adsorption of surfactants, adsorption of macromolecules, esterification, acetylation, acylation, cationisation, silylation, carbamination, molecular grafting, or polymer grafting.

The polymeric matrix in the reinforced nanocomposite of the present disclosure may be made of one or more thermoset or thermoplastic polymers. The polymer may be a biocompatible polymer. Several specific examples of polymers used to form the polymeric matrix, include but are not limited to, a polyester, such as polyethylene terephthalate (PET), polybutylene terephthalate (PBT), polyethylene naphthalate (PEN), or ethylene terephthalate copolymers; a polyamide, such as nylon 6, 6/12, 11, 12, or 66; a polyurethane; a polyethylene; a polyvinyl chloride; a polycarbonate; a poly(meth)acrylate; a maleate; a polyether etherketone (PEEK); a poly(ethylene-co-methacrylic acid) (EMAA); a polyamide/polyether block copolymer; a polyester/polyether block copolymer; a polyamide/polyether polyester copolymer (e.g., Pebax®, Arkema, France); or a PBTpolybutylene oxide block copolymer. Alternatively, the polymeric matrix comprises a polyamide, such as nylon 6 or 12

When desirable other polymeric materials in the form of synthetic or natural rubbers may be used to form the polymeric matrix. These polymeric materials, may include but not be limited to, polyisoprene (synthetic natural rubber), polybutadiene, polychloroprene, butyl rubber, styrenebutadiene rubber, nitrile rubber, hydrogentated nitrile rubber (HNBR), ethylene propylene rubber, ethylene propylene diene rubber (EPDM), chlorosulphonated polyethylene (CSM), chlorinated polyethylene, polysulphide rubber, ethylene acrylic rubber, fluorocarbon rubber, polytetrafluoroethylene-propylene, epichlorohydrin rubber, polyacrylic rubber, silicone rubber, fluorosilicone rubber, polyphosphazene rubber, polyoctenylene, polypropylene oxide rubber, polynorbornene, polyether block amides, EVA rubber, styrenic block copolymers, or other segmented elastomers such as copolyester thermoplastic elastomers (TPEs).

The compatibility between the nanofibers and the polymeric matrix may be enhanced by selecting nanofibers and matrix materials that have compatible functional groups or by functionalizing the surface of the nanofibers to incorporate such compatible groups that are capable of interacting with the matrix material. When desirable, one or more coupling or compatibilizing agents may also be incorporated into the reinforced nanocomposite materials. As used herein, the term(s) coupling or compatibilizing agent includes any agent capable of enhancing the compatibility and/or promoting interaction between the nanofibers and the polymeric matrix material. These compatibilizing agents may be organic or inorganic coupling agents. Several examples of organic coupling agents include, without limitation, amino acids, thiols, maleic anhydride containing polyolefins, and maleimide-functionalized polyamides. Several examples of inorganic coupling agents include, without limitation, alkoxides of silicon, aluminum, titanium, and zirconium, or magnetic powders such as ferrite oxide, to name a few. The amount of the coupling agent added to a nanocomposite material may range from 0.1 wt.% to about 10 wt.% based on the weight of the nanocomposite material; alternatively, from about 0.5 wt.% to about 7.5 wt.%; alternatively, from about 1 wt. % to about 5 wt.% based on the weight of the nanocomposite material.

The reinforced nanocomposite materials may optionally comprise one or more other additives in the form of plasticizers, surfactants or dispersants, stabilizers, pigments, whiteners, conductive agents, magnetic agents, radiopaque agents, therapeutic agents (e.g., drugs), or a pharmaceutically-active compounds.

Inflatable medical balloons have a number of critical design parameters, such as rated burst pressure and tensile strength. The reinforcement of the polymeric matrix with the nanofibers improves the strength and burst pressure of the medical balloon. The inflatable medical balloons of the present disclosure achieve a rated burst pressure (RBP) of at least 15 bar; alternatively, at least 20 bar; alternatively, at least 25 bar; alternatively about 30 bar or greater. The rated burst pressure (RBP) is the statistically determined maximum pressure to which a balloon may be inflated without rupturing. Burst testing of medical balloons may be accomplished using ISO 10555 methodology, according to FDA guidelines, or according to other standard industry protocols.

The medical balloons of the present disclosure may exhibit a tensile strength that is in the range of about 100 MPA to about 500 MPA alternatively, in the range of about 200 MPa to about 400 MPa; alternatively, greater than 100 MPa; alternatively, greater than 200 MPa. The medical balloon may exhibit a maximum tensile strength up to 500 Mpa, up to 450 Mpa, up to 400 MPa, or alternatively, up to 350 Mpa. Tensile testing of medical balloons may be accomplished using ISO 10555 methodology, according to FDA guidelines, or according to other standard industry protocols.

In order to minimize the profile of the delivery system, it may be desirable that the balloon have a low wall thickness (T). However, a trade-off between burst pressure and wall thickness exists because the burst pressure generally decreases when the wall thickness is reduced. The inflatable medical balloons of the present disclosure have a wall thickness (T) that is less than 45 micrometers (µm), less than 35 µm, less than 25 µm, or less than 10 µm. Alternatively, the wall thickness (T) of the nanocomposite materials used to form the inflatable balloon ranges from about 5 micrometers to about 45 micrometers; alternatively, from about 10 micrometers to about 30 micrometers.

The inflatable balloons formed from the reinforced nanocomposite materials of the present disclosure, which comprise nanofibers dispersed and aligned in a polymeric matrix as described above and further defined herein, exhibit enhanced burst pressure and tensile strength as compared to inflatable balloons formed from a composite containing the same polymeric matrix in the absence of any nanofibers. The enhancement in burst pressure and/or tensile strength exhibited by the inflatable balloons of the present disclosure are on the order of about 50% greater, about 25% greater, about 15% greater, about 10% greater, or alternatively, about 5% greater than the burst pressure and/or tensile strength exhibited by a similar balloon made of the same polymeric matrix in the absence of the nanofibers.

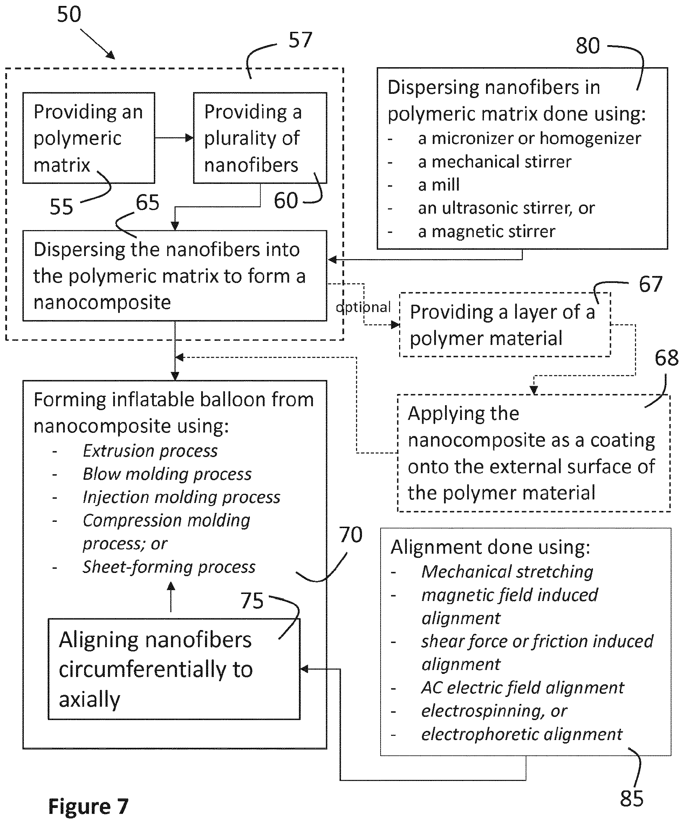

According to another aspect of the present disclosure, a method of forming a medical device comprising an inflatable balloon having an axial length with a wall located along the axial length having an interior surface and an exterior surface that defines a wall thickness there between is provided. Referring now to Figure 7, this method 50 generally comprises providing a polymeric matrix 55, providing a plurality of nanofibers 60, followed by dispersing 65 the nanofibers into the polymeric matrix to form a reinforced nanocomposite material. The inflatable balloon is then formed 70 from the reinforced nanocomposite material using an extrusion process, a blow molding process, an injection molding process, a compression molding process, a sheet-forming process, a combination thereof, or the like. Then the nanofibers are aligned 75 in a predetermined pattern. The predetermined pattern may range from the nanofibers being circumferentially-oriented to axially-oriented relative to the axial length of the inflatable balloon. The alignment 75 of the fibers may be done as part of the forming process 70, for example during extrusion or molding, or the alignment 75 of the fibers may be done in a separate process step.

One or more portions of this process 50 for forming an inflatable balloon may be similar to that used to form a conventional balloon. More specifically, short tubes can be made by extrusion with optional subsequent cutting of the tubes. A small parison may be formed by heating two segments of the tube and then drawing it. The heated segments are separated by a few millimeters to a few centimeters depending on desired parison size. The tube or the parison and most of the drawn tube is placed in a mold capable of applying heating, stretching and internal pressure to the balloon. These steps can be done stepwise or in combination in a defined sequence. The stretching in the mold can that impart axial orientation of fibers and the pressure (inflation) of the parison may impart circumferential orientation. After a balloon has been formed in the mold it is usually heatset (annealed) for a period of time and then cooled.

Compounding of the polymeric matrix may be accomplished in a way that simplifies or reduces the difficulty of dispersing the nanofibers into the polymeric material. According to one aspect of the present disclosure the goal for dispersing 80 the nanofibers into the polymeric matrix is to achieve a homogeneous dispersion of the nanofibers throughout the polymeric matrix. When the nanofiber material comprises a layered material, the dispersion of the nanofiber may involve substantial intercalation; alternatively, exfoliation. As used herein interacalation refers to the reversible inclusion or insertion of a molecule or ion into a material that exhibits a layered structure. Exfoliation represents an extreme case of intercalation in which complete separation of the layers present in the material occurs.

Referring once again to Figure 7, the dispersion 65 of the nanofibers into the polymeric matrix may be a separate step conducted in the manufacturing process 50 or incorporated and performed as part of the polymerization process 57 associated with forming the polymeric matrix material. The nanofibers may be dispersed 80 into the polymeric matrix by mixing the two components together using a micronizer or homogenizer, a mechanical stirrer, a mill, an ultrasonic stirrer, a magnetic stirrer, or the like.

The balloon forming process 70 can be accomplished using conventional methods including, but not limited to, an extrusion process, a blow molding process, an injection molding process, a compression molding process, a sheet-forming process, or the like. The use of an extrusion process may promote isotropy (i.e., same properties in all directions) in the inflatable balloon. Alternatively, axial orientation of the nanofibers in the polymeric matrix may also be promoted by the use of an extrusion process.

Optionally, the reinforced nanocomposite material may be coating that is applied onto another polymer substrate that represents a bulk layer in the inflatable balloon. In this respect, the method 50 may further comprise providing 67 a layer of a polymer material having an external surface and applying 68 the nanocomposite material (i.e., the nanofibers dispersed in the polymeric matrix) as a coating onto the external surface of the polymer layer.

The alignment 85 of the nanofibers in the polymeric matrix may be accomplished using on or more of mechanical stretching of the nanocomposite; magnetic field induced alignment, shear force or friction induced alignment, AC electric field alignment, electrospinning, or electrophoretic alignment, to name a few.

Referring now to Figure 7, the stretching of the nanocomposite material refers to treatment of inflatable balloons 2 as sheets of the reinforced nanocomposite materials 3 and pulling or applying mechanical loads to the sheets in opposed or offset directions. The stretching (see A, B-1 & B-2 in Fig. 7) of the nanocomposite material induces strain in the composite thereby forcing the nanofibers to reorient themselves

(see C-1 & C-2 in Fig. 7). For example, the sheets may be mechanically stretched by passing the sheets between a set of rollers at different speeds, such that one roller is set at a relatively slower rotational speed and "holds" the sheet, while the other roller is set at a relatively faster rotational speed and "stretches" the sheet. The degree of nanofiber alignment may be adjusted by varying the degree of stretching and by varying the temperature at which the stretch takes place. When sheets are stretched, the stretched sheets can be formed into tubes by joining two opposing edges of a sheet.

In a shear force or friction induced alignment process, a polymer nanocomposite with embedded nanofibers is first softened by warming. Then, the softened nanocomposite is unidirectionally rubbed with a blade or a set of parallel grooves, which induces an elastic force that can align the nanotubes, but it also damages the nanocomposite. The advantage of this procedure is that it can be performed automatically.

The use of electric fields to align nanofibers is a technique called electrophoresis. The application of an AC electric field aligns the individual nanofibers between the electrodes. The use of a DC electric field is not suitable as it results in accumulation of nanofibers near one electrode. The use of this technique is sensitive to a combination of several parameters including nanofiber concentration and strength and frequency (kHz-MHz range) of the electric field. The heating of the polymeric matrix to a temperature that is above it's glass transition temperature (T_{g}) is done in order to allow the fibers to rearrange themselves in the polymer matrix. Electrostatic repulsion between charged nanofibers can enhance the alignment of the nanofibers in the reinforced nanocomposite materials.

Magnetic fields may also be utilized to align nanofibers when the nanofibers exhibit magnetic properties. The nanofibers align in the direction of the magnetic field. Contrary to an electric field, which also cause the nanofibers to move, a magnetic field will only reorient the nanofibers.

In addition, the resulting flow of a nanofiber containing polymer mixture into a mold or through an extrusion die may cause the nanofibers contained within the mixture to at least partially align themselves. Aligning the nanofibers either circumferentially or axially results in increased tensile strength, stiffness, and hardness, as well as decreasing brittleness. Injection molding, casting, and hot pressing are examples of processes that are capable of the flow inducement of short nanofiber alignment in a nanocomposite.

For the purpose of this disclosure the terms "about" and "substantially" are used herein with respect to measurable values and ranges due to expected variations known to those skilled in the art (e.g., limitations and variability in measurements).

As used herein, the term "polymer" refers to a molecule having polymerized units of one or more species of monomer. The term "polymer" is understood to include both homopolymers and copolymers. The term "copolymer" refers to a polymer having polymerized units of two or more species of monomers, and is understood to include terpolymers. As used herein, reference to "a" polymer or other chemical compound refers one or more molecules of the polymer or chemical compound, rather than being limited to a single molecule of the polymer or chemical compound. Furthermore, the one or more molecules may or may not be identical, so long as they fall under the category of the chemical compound. Thus, for example, "a" polyurethane is interpreted to include one or more polymer molecules of the polyurethane, where the polymer molecules may or may not be identical (e.g., different molecular weights).

For the purpose of this disclosure, the terms "at least one" and "one or more of an element are used interchangeably and may have the same meaning. These terms, which refer to the inclusion of a single element or a plurality of the elements, may also be represented by the suffix "(s)"at the end of the element. For example, "at least one polyurethane", "one or more polyurethanes", and "polyurethane(s)" may be used interchangeably and are intended to have the same meaning.

Those skilled-in-the-art, in light of the present disclosure, will appreciate that many changes can be made in the specific embodiments which are disclosed herein and still obtain alike or similar result without departing from or exceeding the spirit or scope of the disclosure. One skilled in the art will further understand that any properties reported herein represent properties that are routinely measured and can be obtained by multiple different methods. The methods described herein represent one such method and other methods may be utilized without exceeding the scope of the present disclosure.

Within this specification, embodiments have been described in a way which enables a clear and concise specification to be written, but it is intended and will be appreciated that embodiments may be variously combined or separated without parting from the invention. For example, it will be appreciated that all preferred features described herein are applicable to all aspects of the invention described herein.

The foregoing description of various forms of the invention has been presented for purposes of illustration and description. It is not intended to be exhaustive or to limit the invention to the precise forms disclosed. Numerous modifications or variations are possible in light of the above teachings. The forms discussed were chosen and described to provide the best illustration of the principles of the invention and its practical application to thereby enable one of ordinary skill in the art to utilize the invention in various forms and with various modifications as are suited to the particular use contemplated. All such modifications and variations are within the scope of the invention as determined by the appended claims when interpreted in accordance with the breadth to which they are fairly, legally, and equitably entitled.

## Claims

1. A medical device comprising:
an inflatable balloon having an axial length with a wall located along the axial length having an interior surface and an exterior surface that defines a wall thickness there between; at least a portion of the wall being formed of a reinforced nanocomposite material, the reinforced nanocomposite material comprising:
a polymeric matrix; and
a plurality of nanofibers aligned in a predetermined pattern, wherein the predetermined pattern ranges from the nanofibers being circumferentially-oriented to axially-oriented relative to the axial length of the inflatable balloon.

2. The medical device according to claim 1, wherein the nanofibers are homogenously dispersed throughout the polymeric matrix;
wherein the amount of nanofibers present in the reinforced nanocomposite material ranges from about 0.1 wt. % to less than 5 wt. %, based on the overall weight of the reinforced nanocomposite material.

3. The medical device according to claim 1 or claim 2, wherein the inflatable balloon further comprises a layer of a polymer material that has an external surface, such that the reinforced nanocomposite material is a coating layer located on the external surface of the layer of polymer material.

4. The medical device according to any of claims 1-3, wherein the nanofibers are individually selected to comprise nanofibrillated cellulose (NFC), cellulose nanocrystals (CNC), carbon and ceramic nanotubes, metal oxides, metals, polyacrylonitrile, magnetic or paramagnetic materials, poly(E-caprolactone), poly(lactic acid), poly(glycolic acid), poly(lactide-co-glycolide), poly(L-lactide), poly(L-lactide-co-Ecaprolactone), polyurethane, polyethylene oxide, polyethylene terephthalate, poly (ester urethane)urea, poly[bis(p-methylphenoxy) phosphazene], poly(p-dioxanone-co-L-lactideblock-poly(ethylene glycol), polyvinylpyrrolidone, n-isopropylacrylamide, polysaccharide, polyalkycyanoacrylate, polytetrafluoroethylene, polyamides, collagen, gelatin (denatured collagen), elastin, alpha-elastin, tropoelastin, and chitosan..

5. The medical device according to any of claims 1-4, wherein the nanofibers further comprise a surface treatment that forms hydrophilic groups, hydrophobic groups, or a combination thereof on the external surface of the nanofibers.

6. The medical device according to any of claims 1-5, wherein the polymeric matrix comprises cellulose, polyamide, polyethylene terephthalate (PET), polybutylene terephthalate (PBT), polyethylene naphthalate (PEN), and ethylene terephthalate copolymers; polyurethane; polyethylene; polyvinyl chloride; polycarbonate; poly(meth)acrylate; maleate; polyether etherketone (PEEK); poly(ethylene-co-methacrylic acid) (EMAA); polyamide/polyether block copolymer, polyester/polyether block copolymer; polyamide/polyether polyester copolymer; PBT polybutylene oxide block copolymer or a combination thereof.

7. The medical device according to any of claims 1-6, wherein the inflatable balloon comprises two or more layers of the reinforced nanocomposite material;
wherein the nanofibers present in one layer are oriented in a different direction than the nanofibers in another layer.

8. The medical device according to any of claims 1-7, wherein the nanofibers are oriented biaxially at an intermediate angle that is between the nanofibers being axially-oriented and circumferentially-oriented with respect to the axial length of the inflatable balloon.

9. The medical device according to any of Claims 1-8, wherein the inflatable balloon further comprises at least one of an inner protective layer and an outer protective layer.

10. The medical device according to any of claims 1-9, wherein the nanofibers have a length that ranges from about 5 nanometers (nm) up to about 50,000 nm and a diameter that ranges from about 1 nanometer to about 20 nanometers, preferably wherein the nanofibers exhibit an aspect ratio that ranges from 25 to about 5,000.

11. The medical device according to any one of claims 1-10, wherein:
the majority or substantially all of the nanofibers are oriented orthogonal to the direction of wall thickness; and/or
the nanofibers in a portion of the wall extending 360° about the axial length of the balloon are oriented predominantly with a common orientation direction or oriented predominantly with one of only two common orientation directions, for that portion.

12. A method of forming a medical device comprising an inflatable balloon having an axial length with a wall located along the axial length having an interior surface and an exterior surface that defines a wall thickness there between, the method comprising:
providing an polymeric matrix;
providing a plurality of nanofibers;
dispersing the plurality of nanofibers into the polymeric matrix to form a reinforced nanocomposite material;
forming the inflatable balloon from the reinforced nanocomposite material using an extrusion process, a blow molding process, an injection molding process, a compression molding process, or a sheet-forming process; and
aligning the nanofibers in a predetermined pattern, wherein the predetermined pattern ranging from the nanofibers being circumferentially-oriented to axially-oriented relative to the axial length of the inflatable balloon.

13. The method according to Claim 12, wherein the alignment of the fibers is accomplished using on or more of mechanical stretching of the nanocomposite; magnetic field induced alignment, shear force or friction induced alignment, AC electric field alignment, electrospinning, and electrophoretic alignment.

14. The method according to any of claims 12 or 13, wherein the method further comprises providing a layer of a polymer material having an external surface; and applying the reinforced nanocomposite material as a coating layer onto the external layer of the polymer material.

15. The use of a reinforced nanocomposite material to form a medical device that includes an inflatable balloon having an axial length with a wall located along the axial length having an interior surface and an exterior surface that defines a wall thickness there between; at least a portion of the wall being formed of the reinforced nanocomposite material, the reinforced nanocomposite material comprising:
a polymeric matrix; and
a plurality of nanofibers aligned in a predetermined pattern, wherein the predetermined pattern ranges from the nanofibers being circumferentially-oriented to axially-oriented relative to the axial length of the inflatable balloon.
